# EUROPÄISCHE PATENTSCHRIFT

(11) **EP 3 545 907 B1**
(45) Veröffentlichungstag und Bekanntmachung des Hinweises auf die Patenterteilung: **26.04.2023**
(21) Anmeldenummer: 18163947.7
(22) Anmeldetag: 26.03.2018
(51) Int. Cl.: A61F 2/30, A61L 27/54, A61M 31/00

(54) **WIRKSTOFFAPPLIKATIONSVORRICHTUNG FÜR EIN GELENKIMPLANTAT UND GELENKIMPLANTAT MIT EINER WIRKSTOFFAPPLIKATIONSVORRICHTUNG**
ACTIVE INGREDIENT APPLICATION DEVICE FOR A JOINT IMPLANT AND JOINT IMPLANT WITH ACTIVE INGREDIENT APPLICATION DEVICE
DISPOSITIF D'APPLICATION DE SUBSTANCES ACTIVES POUR UN IMPLANT D'ARTICULATION ET IMPLANT D'ARTICULATION DOTÉ D'UN TEL DISPOSITIF D'APPLICATION DE SUBSTANCES ACTIVES

(43) Veröffentlichungstag der Anmeldung: 02.10.2019
(73) Patentinhaber: Waldemar Link GmbH & Co. KG, 22339 Hamburg (DE)
(72) Erfinder: CAPANNA, Rodolfo, 50139 Firenze (IT); LINK, Helmut D., 22397 Hamburg (DE)
(74) Vertreter: AWA Denmark A/S

(56) Entgegenhaltungen:
- EP-A1- 0 244 720
- EP-A1- 2 052 700
- EP-A2- 2 328 511
- US-A1- 2004 180 072
- US-A1- 2010 042 214
- US-A1- 2015 250 598

## Beschreibung

### GEBIET DER ERFINDUNG

Das Patent betrifft einen Wirkstoffapplikationseinsatz für ein Implantat, insbesondere ein Gelenkimplantat, und ein Implantat mit solch einem Wirkstoffapplikationseinsatz.

### STAND DER TECHNIK

Eine Implantation ist bei Gelenkimplantaten, insbesondere bei einem vollständigen Kniegelenkersatz, durch die Größe dieser Implantate bedingt, ein vergleichsweise großer Eingriff. Folglich wird bei einem solchen Eingriff ein Implantat in den Körper eines Patienten eingebracht, das eine entsprechend große Fläche aufweist. Diese Implantatfläche ist vor der Implantation der Umgebung außerhalb des Patienten ausgesetzt. Bereits deswegen kann am Eingriffsort keine absolute Sterilität sichergestellt werden, die notwendig wäre, um eine periprothetische Infektion mit an Sicherheit grenzender Wahrscheinlichkeit ausschließen zu können. Neben hygienischen Risikofaktoren gibt es zudem auch patientenspezifische Risikofaktoren, die eine periprothetische Infektion begünstigen können.

Kommt es zu so einer periprothetischen Infektion, wird diese im Allgemeinen durch Verabreichung von Wirkstoffen, insbesondere von Antibiotika, bekämpft. Gerade bei Risikopatienten kann eine solche Behandlung auch vorbeugend durchgeführt werden. Die Wirkstoffe werden direkt am Implantationsort freigesetzt. Hierfür kommen zum Beispiel Kollagenschwämme, Vliese oder Ketten mit Knochenzementkügelchen zum Einsatz, in denen sich ein Wirkstoff befindet, der nach und nach freigegeben wird, um Entzündungen zu bekämpfen oder gegen diese vorzubeugen.

Bei anderen Verabreichungstechniken wird der Wirkstoff von außen über Infusionsschläuche zum Gelenk geführt und dort verabreicht. Es können auch zusätzliche, einen Wirkstoff tragende Implantate implantiert werden. Diese weisen auf ihrer Oberfläche Aussparungen auf, in denen ein Wirkstoff eingebracht ist. Der Wirkstoff wird dann im implantierten Zustand im Körper nach und nach freigegeben. So offenbart zum Beispiel die EP 2 052 700 A1 ein Wirkstoffabgabeimplantat, das zusammen mit einer Gelenkendoprothese implantiert wird und derartige Aussparungen mit einem Wirkstoff aufweist.

Gelingt es nicht, die Infektion durch eine alleinige Verabreichung von Wirkstoffen zurückzudrängen, kann es notwendig werden, einen Teil oder sogar das ganze Implantat wieder herauszunehmen. Dabei kommt es allerdings zu einer weitgehenden Immobilisierung des Patienten. Um dem zu begegnen, können anstelle des entnommenen Implantats Platzhalterimplantate eingesetzt werden. Hierzu gehören zum Beispiel Implantatimitationen aus Knochenzement, wobei der Knochenzement ebenfalls mit einem Wirkstoff angereichert ist. Durch diese Platzhalterimplantate wird versucht, der immobilisitationsbedingten Weichteilkontraktur, Muskelatrophie und Knochenchwächung entgegen zu wirken. Allerdings ist dies nur beschränkt möglich.

Alternativ gibt es Implantate, an denen von Beginn an Wirkstoffaussparungen vorgesehen sind. So offenbart die US 4,919,666 A eine Hüftendoprothese und eine Hüftpfanne, in denen Taschen vorgesehen sind, die bei Implantation ebenfalls einen Wirkstoff enthalten. Die US 2004/0180072 A1 schlägt zu dem gleichen Zweck einzelne vergleichsweise große Sacklöcher in Implantaten vor, um das Gelenk mit Wirkstoffen zu versorgen.

Hierbei ergibt sich allerdings das Problem, dass, einmal implantiert, die Wirkstoffabgabe nicht mehr aufgehalten werden kann. Zudem kann der Wirkstoff nicht gewechselt werden, da er in einer resorbierbaren Substanz in den Wirkstoffaussparungen vergossen ist, diese also auffüllt. Beides kann allerdings notwendig werden, wenn der Patient eine Überempfindlichkeit gegen den Wirkstoff zeigt. Als Folge kann es notwendig sein, das Implantat wieder zu explantieren, da der Wirkstoff nicht anders entnommen, geschweige denn gewechselt werden kann. Auch unterliegt die Wirksamkeit der verwendeten Wirkstoffe einer zeitlich beschränkten Haltbarkeit.
WO 2010/025378 A2 offenbart ein in einen Körper implantierbares orthopädisches Implantat, das mindestens einen therapeutischen Wirkstoff in den Körper einbringen kann. Das Implantat weist ein Reservoir und eine Vielzahl von Kanälen auf. Das Reservoir ist für die Aufnahme des mindestens einen therapeutischen Wirkstoffs eingerichtet und die Vielzahl von Kanälen befördern den therapeutischen Wirkstoff dann aus dem Reservoir zu der Behandlungsstelle im Körper. Zudem kann eine Wirkstoffpatrone verwendet werden, welche den therapeutischen Wirkstoff in das Reservoir und die Kanäle abgibt.

All dies macht das Herstellen, Vorhalten und Lagern von Implantaten mit integrierten Wirkstoffen kompliziert und damit unhandlich. Weiterhin können Wirkstoffaussparungen den Nachteil aufweisen, dass sie nach Abgabe des Wirkstoffs Vertiefungen an der Oberfläche des Implantats zurücklassen. Durch ihre Funktion, eine ausreichend große Menge an Wirkstoffen vorzuhalten, sind diese Aussparungen für das Einwachsen von umliegendem wenig geeignet. Gerade wenn die Vertiefungen in einem Bereich vorgesehen sind, der mit Knochengewebe in Kontakt kommt, wächst dieses nicht ein. Als Folge steht die dortige Fläche dieser Aussparungen für eine Verankerung des Implantats nicht zur Verfügung. Falls diese Aussparungen in einem Bereich des Implantats liegen, der mit Weichteilgewebe in Kontakt kommt, kann es durch die zurückbleibenden Aussparungen ebenfalls zu Komplikationen kommen. Ein diesbezügliches Beispiel ist eine lokale Ansammlung fibrösen Gewebes, welche die Funktion des Gelenks negativ beeinflussen kann.

### ZUSAMMENFASSUNG DER ERFINDUNG

Vor diesem Hintergrund war es eine Aufgabe der vorliegenden Erfindung, einen Wirkstoffapplikationseinsatz bereitzustellen, mit dem nicht nur eine Wirkstoffapplikation sondern auch ein Wirkstoffwechsel oder eine gezielte Unterbrechung der Wirkstoffabgabe möglich ist. Zudem soll die Anzahl der Eingriffe zur Verabreichung des Wirkstoffs möglichst gering gehalten werden.

Angesichts dieser Aufgaben stellt die Erfindung einen Wirkstoffapplikationseinsatz für ein Implantat, insbesondere ein Gelenkimplantat, bereit. Der Wirkstoffapplikationseinsatz weist einen Grundkörper, eine in dem Grundkörper ausgebildete Wirkstoffkammer und eine Applikationsseite auf. Die Applikationsseite bildet eine Vorderseite der Wirkstoffkammer aus, wobei die Applikationsseite mehrere Applikationsöffnungen einschließt. Zudem weist der Wirkstoffapplikationseinsatz ein Befestigungsmittel auf, mit dem der Grundkörper abnehmbar in dem Implantat befestigbar ist. In einem Zustand, in dem der Grundkörper in das Implantat eingesetzt ist, ist die Applikationsseite relativ zu dem Implantat nach außen gewandt, sodass die Applikationsöffnungen die Wirkstoffkammer mit der Umgebung des Implantats verbinden.

Durch diesen Aufbau ist der Wirkstoffapplikationseinsatz ein in das Gelenkimplantat integrierbarer Bestandteil des Implantats. Folglich handelt es sich bei dem Wirkstoffapplikationseinsatz um ein zusätzliches Teil, das implantiert wird, ohne dass es am Implantationsort zusätzlichen Raum einnimmt.

Weiterhin ist es durch das Befestigungsmittel möglich, einen einmal eingesetzten Wirkstoffapplikationseinsatz wieder zu entnehmen, um zum Beispiel den Wirkstoff aufzufüllen, zu wechseln oder zu entnehmen. Dies ist vor allem bei einer auftretenden Empfindlichkeit des Wirkstoffs beim Patienten von Vorteil, da hierdurch eine Explantation verhindert werden kann.

Weiterhin ist durch eine direkte Platzierung des Wirkstoffapplikationseinsatzes im Implantat gewährleistet, dass der in der Wirkstoffkammer enthaltene Wirkstoff direkt in die periprophetische Umgebung freigegeben wird. Hierdurch kann insbesondere einem Biofilm auf der Oberfläche des Implantats vorgebeugt werden, der durch seinen schützenden Effekt für Pathogene die Entstehung einer Infektion begünstigen kann.

Die nach außen gewandte Applikationsseite mit den darin ausgebildeten Applikationsöffnungen bildet im eingesetzten Zustand zumindest einen Teil der Außenfläche des Implantats aus. Dabei ist die als Stirnwand ausgebildete Applikationsseite bevorzugt mit der Außenseite des Implantats bündig. Noch bevorzugter fügt sie sich geometrisch in das äußere Erscheinungsbild des Implantats, d. h. dessen Kontur, ein.

Bei einer bevorzugten Ausführungsform ist auf der zu der Applikationsseite gegenüberliegenden Seite eine Rückwand ausgebildet, welche eine Rückseite der Wirkstoffkammer ausbildet und in dem eingesetzten Zustand dem Implantat zugewandt ist.

Weist die Wirkstoffkammer eine Rückwand auf, so ist die Wirkstoffkammer ein mit Ausnahme der Applikationsöffnungen abgeschlossener Raum. Der Wirkstoff kann somit nur über die Applikationsöffnungen entweichen.

Bei einer weiteren bevorzugten Ausführungsform ist zumindest ein Abschnitt der Rückwand auf der Rückseite der Wirkstoffkammer durch einen vorzugsweise wiederverschließbaren Deckel ausgebildet.

Durch Vorsehen und Anordnen eines Deckels auf der Rückseite der Wirkstoffkammer kann sichergestellt werden, dass sich ein falsch eingesetzter Deckel oder wiederverschließbarer Deckel unbeabsichtigt öffnet, da er in einem im Implantat eingesetzten Zustand dem Boden der Aussparung im Implantat zugewandt ist und dadurch verschlossen gehalten wird.

Vorzugsweise wird der Deckel über einen Reibschluss oder mittels eines Snap-Fits oder Formschlusses an dem Wirkstoffapplikationseinsatz befestigt. Weiterhin bildet der Deckel vorzugsweise im Wesentlichen die Rückseite der Wirkstoffkammer aus.

Bei einer bevorzugten Ausführungsform weist der Grundkörper zwischen der Applikationsseite und der Rückseite eine umlaufende Außenwand auf, die zylindrisch, insbesondere mit einem kreisförmigen Querschnitt, ausgebildet ist und die Wirkstoffkammer umgibt.

Durch die zylindrische umlaufende Außenwand des in ein Implantat einsetzbaren Grundkörpers ist ein einfaches Einsetzen entlang einer Einsetzrichtung in das Implantat möglich. Mit anderen Worten kann der Wirkstoffapplikationseinsatz auf einfache Weise durch eine translatorische Bewegung entlang der Einsetzrichtung eingesetzt und wieder entnommen werden.

Weiterhin ist es möglich, die zylindrische Form mit einem kreisförmigen Querschnitt zu versehen, sodass ein Einsetzen des Wirkstoffapplikationseinsatzes unabhängig von dessen Orientierung um die Längsachse des Grundkörpers möglich ist. Diese Längsachse stimmt vorzugsweise mit einer Einsetzrichtung überein.

Bei einer weiteren bevorzugten Ausführungsform bildet zumindest ein Abschnitt der Außenseite der umlaufenden Außenwand eine Reibfläche als Befestigungsmittel aus.

Die Verwendung einer Reibfläche und damit eines Reibschlusses zum Befestigen des eingesetzten Wirkstoffapplikationseinsatzes stellt eine einfache Handhabbarkeit bereit und ermöglicht zudem, falls erforderlich oder gewünscht, eine Entnahme des Wirkstoffapplikationseinsatzes.

Bei einer weiteren Ausführungsform bildet die umlaufende Außenwand zumindest einen Abschnitt einer umlaufenden Wirkstoffkammerwand zwischen der Applikationsseite und der Rückseite der Wirkstoffkammer aus.

Dadurch, dass die umlaufende Außenwand zumindest einen Abschnitt einer umlaufenden Wirkstoffkammerwand ausbildet, kann das Volumen der Wirkstoffkammer und somit ein für den Wirkstoff zur Verfügung stehendes Aufnahmevolumen maximiert werden. Für den Fall, dass die umlaufende Außenwand die gesamte umlaufende Wirkstoffkammerwand ausbildet, ergibt sich zudem ein sehr einfacher und damit kostengünstig herstellbarer Aufbau des Wirkstoffapplikationseinsatzes.

Bei einer besonders bevorzugten Ausführungsform ist zumindest ein Abschnitt der umlaufenden Wirkstoffkammerwand zwischen der Applikationsseite und der Rückseite der Wirkstoffkammer getrennt von und innenliegend zu der umlaufenden Außenwand des Grundkörpers ausgebildet, sodass entlang dieses Abschnitts zwischen der Außenwand des Grundkörpers und der Wirkstoffkammerwand ein Spalt ausgebildet ist.

Diese Ausführungsform hat den Vorteil, dass zumindest ein Abschnitt der Wirkstoffkammer in dem Grundkörper des Wirkstoffapplikationseinsatzes durch ihre Anordnung räumlich von der umlaufenden Außenwand des Grundkörpers getrennt ist. Damit können der Grundkörper und die Wirkstoffkammer in diesem Abschnitt auch funktional voneinander getrennt werden. Insbesondere kann in diesem Abschnitt das Befestigungsmittel an der Außenseite der umlaufenden Außenwand des Grundkörpers vorgesehen sein. Durch den Spalt, der vorzugsweise wie die Applikationsöffnungen relativ zu dem eingesetzten Wirkstoffapplikationseinsatz nach außen exponiert ist, kann eine Elastizität des Befestigungsmittels zuverlässig und einfach vorbestimmt Zurück werden. Weist das Befestigungsmittel zum Beispiel eine Reibfläche auf, kann die durch die Reibfläche auf das Implantat ausgeübte Reibkraft über die Elastizität eingestellt werden, sodass der Wirkstoffapplikationseinsatz in dem im Implantat eingeführten Zustand an Ort und Stelle befestigt ist. Weiterhin kann durch die Einstellung der Elastizität ein einfaches und störungsfreies Einsetzen und Entnehmen des Wirkstoffapplikationseinsatzes gewährleistet werden.

Durch die räumliche Trennung bildet der Abschnitt der umlaufenden Außenwand des Grundkörpers, der von der umlaufenden Wirkstoffkammerwand getrennt ist, einen vorzugsweise ringförmigen Körper, mit dem der Wirkstoffapplikationseinsatz in dem Implantat befestigt werden kann.

Durch diese räumliche Trennung ist auch die umlaufende Außenwand der Wirkstoffkammer zumindest abschnittsweise von der umlaufenden Außenwand des Grundkörpers getrennt und damit von etwaigen Druckkräften, die durch das Befestigungsmittel im eingesetzten Zustand des Wirkstoffapplikationseinsatzes vorhanden sind, befreit. Als Folge kann einer möglichen Beschädigung der Wirkstoffkammer beim Einsetzen vorgebeugt werden, die sich ansonsten auf ungewünschte Weise auf die Wirkstoffabgabe auswirken kann.

Für den Fall, dass die umlaufende Außenwand des Grundkörpers und die umlaufende Außenwand der Wirkstoffkammer über den gesamten Bereich zwischen der Applikationsseite und der Rückseite der Wirkstoffkammer getrennt ist, sind die beiden umlaufenden Außenwände vorzugsweise durch die Rückwand der Wirkstoffkammer miteinander verbunden. Mit anderen Worten stehen sie bei einer solchen Ausführungsform als Vorsprünge von der Rückwand der Wirkstoffkammer hervor. Dementsprechend erstreckt sich die Rückwand über den äußeren Umfang der Wirkstoffkammer hinaus.

Bei einer bevorzugten Ausführungsform ist die Applikationsseite mit einem Werkzeugeingriffsmittel zum Einsetzen und/oder Herausnehmen des Wirkstoffapplikationseinsatzes versehen.

Mit dem Werkzeugeingriffsmittel kann der Wirkstoffapplikationseinsatz auf einfache Weise in das Implantat eingesetzt und insbesondere auch wieder entfernt werden. Das Werkzeugeingriffsmittel ist dementsprechend vorzugsweise an der nach außen gewandten Vorderseite des Wirkstoffapplikationseinsatzes bzw. der Wirkstoffkammer vorgesehen. Des Weiteren ist das Werkzeugeingriffsmittel vorzugsweise mittig und/oder am äußeren Rand der Vorderseite vorgesehen.

Bei einer besonders bevorzugten Ausführungsform ist das Werkzeugeingriffsmittel als Aussparung ausgebildet ist, die vorzugsweise ein Innengewinde aufweist.

Durch Vorsehen einer Aussparung als Werkzeugeingriffsmittel kann eine Verletzung umliegenden Körpergewebes durch das Werkzeugeingriffsmittel im implantierten Zustand verhindert werden. Zudem kann das Werkzeugeingriffsmittel einfach gefunden werden und gibt dem Benutzer insbesondere bei einer Entnahme des Wirkstoffapplikationseinsatzes durch die Aufnahme des Werkzeugs ein direktes Feedback, dass das Werkzeug korrekt positioniert ist. Ist zudem ein Innengewinde vorgesehen, kann einem Abrutschen des Wirkstoffapplikationseinsatzes sowohl beim Einsetzen in das Implantat als auch beim Herausnehmen des Wirkstoffapplikationseinsatzes aus dem Implantat zuverlässig vorgebeugt werden.

Bei einer bevorzugten Ausführungsform weist die Applikationsseite mindestens 4, 6, 8 oder 10 und maximal 30 Applikationsöffnungen auf, die vorzugsweise gleichmäßig über die Applikationsseite verteilt sind.

Durch eine entsprechende Auswahl der Applikationsöffnungen kann somit die Dosierung des Wirkstoffes optimal ausgewählt werden. Zudem kann über eine entsprechende Verteilung über die Applikationsseite in einem gewissen Umfang die örtliche Verabreichung des Wirkstoffes gesteuert werden.

Weiterhin weist der Grundkörper des Wirkstoffapplikationseinsatzes vorzugsweise einen Querschnitt mit einer Größe von 0,5 cm bis 3 cm und bevorzugt zwischen 1 cm und 3 cm auf.

Bei einer weiteren Ausführungsform weisen die Applikationsöffnungen eine Größe von mindestens 0,5 mm oder 1 mm und maximal 2 mm, 3 mm oder 4 mm auf.

Auch die Größe der Applikationsöffnungen ermöglicht eine optimale Einstellung der Dosierung des Wirkstoffs. Zudem können auch unterschiedlich große Applikationsöffnungen bei einer Applikationsseite vorgesehen sein, um die Wirkstoffabgabe in Abhängigkeit davon, wo sich eine Applikationsöffnung befindet, zu steuern. Zum Beispiel können Applikationsöffnungen im mittigen Bereich der Applikationsseite einen größeren Durchmesser aufweisen, da der Wirkstoff von diesem Bereich aus tendenziell in alle Richtungen diffundiert und sich somit stärker verteilt, während er im Randbereich der Applikationsseite vor allem nach außen diffundiert.

Bei asymmetrischen Öffnungsquerschnitten bezieht sich die Größe auf den größten Öffnungsbereich einer Applikationsöffnung.

Bei einer bevorzugten Ausführungsform sind die Applikationsöffnungen kreisförmig, oval, rechtwinklig und/oder schlitzförmig ausgeführt.

Die Applikationsöffnungen können zudem die Applikationsseite gleichmäßig verteilt perforieren. Weiterhin ist es möglich, dass auf der Applikationsseite zumindest abschnittsweise ein Gitter und/oder ein Membran vorgesehen ist, um den in der Wirkstoffkammer enthaltenen Wirkstoff im implantierten Zustand des Wirkstoffapplikationseinsatzes in das umliegende Gewebe abzugeben.

Bei einer bevorzugten Ausführungsform weist die Wirkstoffkammer mindestens einen Wirkstoff, insbesondere ein Antibiotikum, auf. Dabei liegt der Wirkstoff in Form eines die Wirkstoffkammer zumindest teilweise ausfüllenden Feststoffs, Kugeln, Granulats und/oder Gels vor.

Folglich kann der mindestens eine Wirkstoff auf vielfältige Weise in der Wirkstoffkammer aufgenommen werden. Allerdings liegt er vorzugsweise so vor, dass er nicht nur durch die Querschnittsverengung der Applikationsöffnungen über einen Zeitraum abgegeben wird, sondern selbst in einer Form, zum Beispiel zusammen mit Hilfsstoffen, vorliegt, die zu einer verlangsamten Abgabe des Wirkstoffs an das umliegende Gewebe führt.

Zudem stellt die vorliegende Erfindung ein Implantat, insbesondere ein Gelenkimplantat und noch bevorzugter ein Kniegelenkimplantat, bereit, das mindestens eine Aussparung aufweist, die für eine Aufnahme eines Wirkstoffapplikationseinsatzes nach einem der vorstehenden Ansprüche ausgeführt ist.

Dabei ist es möglich, anstelle eines Wirkstoffapplikationseinsatzes einen geometrisch dementsprechenden Körper ohne Applikationsöffnungen vorzusehen, der die Aussparung des Implantats ausfüllt, falls keine Abgabe bzw. Verabreichung eines Wirkstoffs notwendig ist. Ein solcher Körper kann dabei eine nach außen weisende beschichtete Oberfläche aufweisen, die zum Beispiel das Anwachsen umliegenden Gewebes ermöglicht. Dies ist insbesondere von Vorteil, wenn die mindestens eine Aussparung in einem Bereich vorgesehen ist, der im implantierten Zustand Knochengewebe zugewandt ist.

Der Wirkstoffapplikationseinsatz oder ein entsprechender Ersatzkörper ermöglichen jeweils, dass anstelle von Ersatzkörpern oder Interimsimplantaten ein dauerhaftes Implantat mit einem Wirkstoff versorgt werden kann. Damit wird einer anderweitig notwendigen Explantation und daraus resultierender Immobilisation des Patienten vorgebeugt.

Weiterhin sind bevorzugt maximal vier Aussparungen in dem Implantat vorhanden und noch bevorzugter ein bis zwei Aussparungen, um eine entsprechende Anzahl von Wirkstoffapplikationseinsätzen aufnehmen zu können. Insbesondere sind bei einer Femurkomponente eines Kniegelenkimplantats zwei derartige Aussparungen vorgesehen.

### KURZE BESCHREIBUNG DER FIGUREN

In den diese Beschreibung ergänzenden Figuren werden beispielhafte Ausführungsformen der vorliegenden Erfindung veranschaulicht und beschrieben. Dabei wird auf einzelne Merkmale dieser beispielhaften Ausführungsformen mittels Bezugszeichen verwiesen, wobei sich gleiche Bezugszeichen über die Figuren hinweg auf Merkmale beziehen, welche gleich ausgebildet und/oder eine gleiche Funktion aufweisen.
Figur 1 veranschaulicht eine erste Ausführungsform eines Wirkstoffapplikationseinsatzes von der Applikationsseite aus, zusammen mit einem abgenommenen Deckel, der für die Rückseite der Wirkstoffkammer vorgesehen ist.
Figur 2 ist eine Seitenansicht des Wirkstoffapplikationseinsatzes aus Figur 1, ebenfalls zusammen mit dem abgenommenen Deckel.
Figur 3 gibt schräg von der Rückseite des Wirkstoffapplikationseinsatzes aus einen Einblick in die Wirkstoffkammer des Wirkstoffapplikationseinsatzes aus Figur 1.
Figur 4 veranschaulicht ein Implantat schräg von anterior mit einem eingesetzten Wirkstoffapplikationseinsatz gemäß einer zweiten beispielhaften Ausführungsform.

### AUSFÜHRLICHE BESCHREIBUNG BEVORZUGTER AUSFÜHRUNGSFORMEN

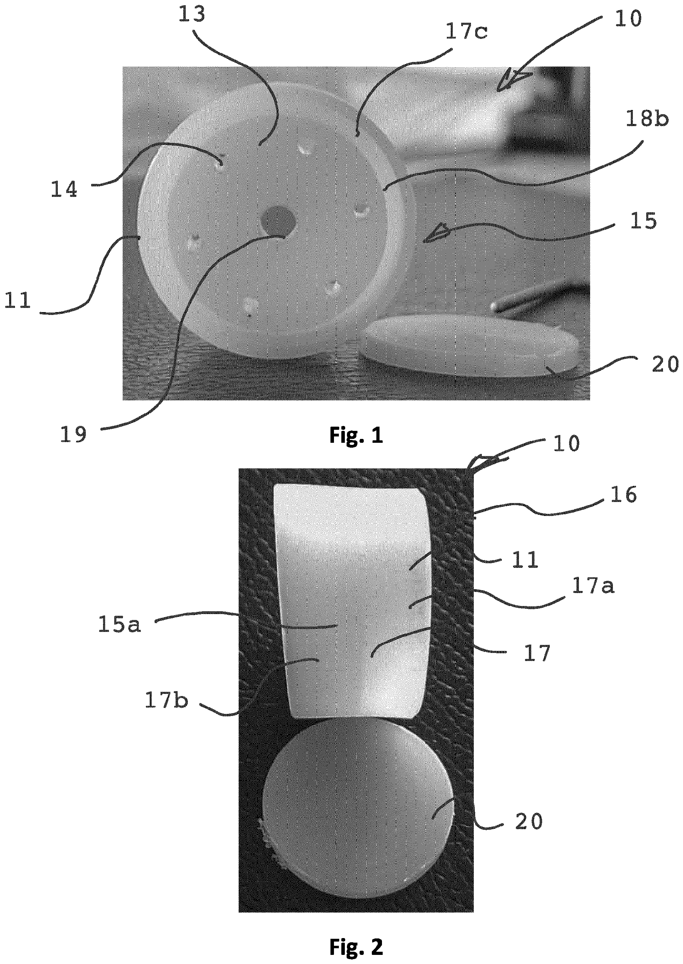

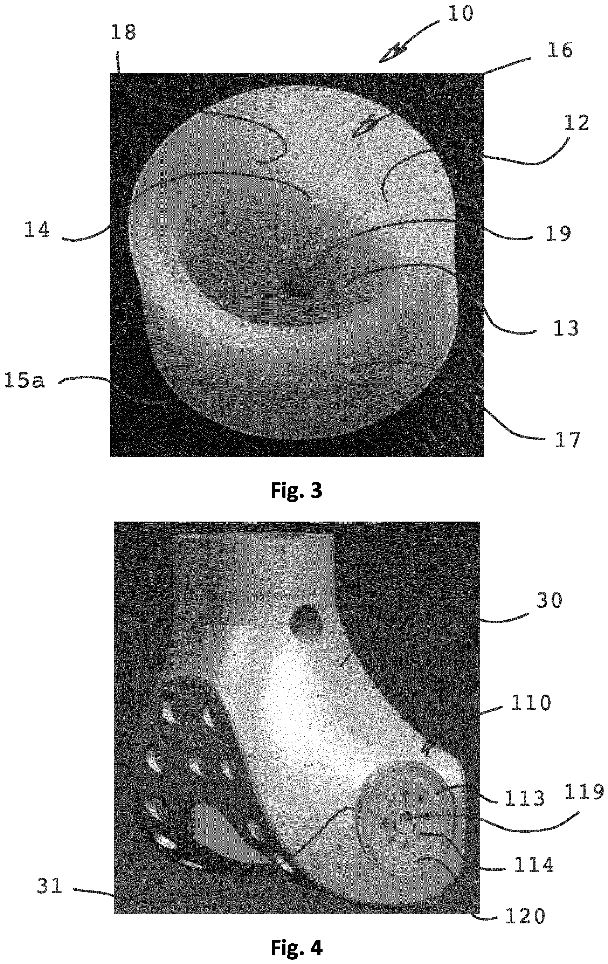

Auf der linken Seite der Figur 1 wird ein Wirkstoffapplikationseinsatz 10 von dessen Applikationsseite 13 veranschaulicht. Die Applikationsseite 13 ist als Applikationsstirnwand ausgebildet. Rechts neben dem Wirkstoffapplikationseinsatz 10 ist in Figur 1 eine als abnehmbarer Deckel 20 ausgeführte Rückseite 16 dargestellt. Der abnehmbare Deckel 20 kann in eine ringförmige Aussparung 16a der Rückseite 16 gedrückt werden (siehe Figur 3), sodass er durch einen Reibschluss in der ringförmigen Aussparung 16a gehalten wird und in diesem Zustand die Rückseite 16 der Wirkstoffkammer 12 verschließt.

Der Deckel 20 ist bei der gezeigten Ausführungsform auf der Rückseite des Wirkstoffapplikationseinsatzes 10 vorgesehen, die dem Boden einer entsprechenden Aussparung 31 in einem Implantat 30 zugewandt ist. Damit wird einer unbeabsichtigten Öffnung des Deckels 20 effektiv vorgebeugt. Abweichend von der gezeigten Ausführungsform eines Wirkstoffapplikationseinsatzes 10 ist es auch möglich, anstelle eines Deckels 20 eine feste Rückwand auf der Rückseite 16 vorzusehen. Allerdings erleichtert eine als Wirkstoffkammerdeckel 20 ausgeführte Rückseite 16 das Befüllen des Wirkstoffapplikationseinsatzes 10 mit einem Wirkstoff, der in einer der oben bereits beschriebenen Formen vorliegen kann. Es abweichend davon auch möglich, ganz auf eine Rückwand auf der Rückseite 16 zu verzichten. Bei einer solchen Ausführungsform verschließt der Boden einer Aussparung 31 in einem Implantat 30 die Rückseite 16 des Wirkstoffapplikationseinsatzes 10 in dessen eingesetztem Zustand.

In der Applikationsseite 13 sind Applikationsöffnungen 14 ausgebildet. Über diese Applikationsöffnungen 14 kommuniziert die Wirkstoffkammer 12 mit der Außenseite des Wirkstoffapplikationseinsatzes und damit im implantierten Zustand des Wirkstoffapplikationseinsatzes 10 mit dem Körpergewebe eines Patienten. Dabei kann die Applikationsseite Knochengewebe oder Weichteilgewebe zugewandt sein. Vorzugsweise wird der Wirkstoffapplikationseinsatz 10 bei einem Gelenkimplantat und noch bevorzugter bei einem Kniegelenkersatz eingesetzt (vergleiche Figur 4).

Die in Figur 1 dargestellte Applikationsseite 13 weist als Applikationsöffnungen 14 sechs kreisförmige Durchgangslöcher auf. Wie oben bereits beschrieben, können alle oder einige der Applikationsöffnungen 14 auch einen anderen Querschnitt als einen kreisförmigen Querschnitt aufweisen. Die Anzahl und/oder Größe der Applikationsöffnungen kann entsprechend der gewünschten Dosierung angepasst werden. Im Allgemeinen gilt, dass eine größere Anzahl von Applikationsöffnungen 14 und/oder eine Vergrößerung der Applikationsöffnungen 14 die Dosierung eines in der Wirkstoffkammer 12 enthaltenen Wirkstoffs ansteigen lässt.

Weiterhin ist es möglich, in zumindest einem Abschnitt der Applikationsseite 14 eine Membran, ein textiles Material und/oder ein Gitter vorzusehen. Bei einer Membran und/oder einem Textil ist es zudem möglich, die Durchlässigkeit für den Wirkstoff von der Wirkstoffkammer 12 aus nach außen und/oder die Durchlässigkeit von Körperflüssigkeit in die Wirkstoffkammer gezielt einzustellen. Hierdurch kann der Applikationszeitraum für den Wirkstoff zusätzlich gesteuert werden.

Bei der in Figur 1 gezeigten beispielhaften Ausführungsform weist der Wirkstoffapplikationseinsatz 10 zudem ein Werkzeugeingriffsmittel 19 auf. Dieses ist bei dieser Ausführungsform als Durchgangsloch ausgeführt und weist ein Innengewinde auf. Zudem ist aus der Figur 3 ersichtlich, dass das Werkzeugeingriffsmittel bei dieser beispielhaften Ausführungsform zudem eine Verbindung zwischen der Wirkstoffkammer 12 des Wirkstoffapplikationseinsatzes 10 und der Außenseite des Wirkstoffapplikationseinsatzes bereitstellt. Folglich bildet das Werkzeugeingriffsmittel bei dieser Ausführungsform eine Applikationsöffnung aus.

Wie allerdings bei einer in Figur 4 dargestellten zweiten beispielhaften Ausführungsform eines Applikationseinsatzes 110 zu erkennen ist, kann das Werkzeugeingriffsmittel 119 auch als ein Durchgangsloch oder Sackloch ausgeführt sein, welches so in den Wirkstoffapplikationseinsatz 110 eingebracht ist, dass es keine Verbindung zur Wirkstoffapplikationskammer 112 bereitstellt. Dazu ist bei der beispielhaften Ausführungsform der Figur 4 eine mittige Säule in der Wirkstoffkammer 112 vorgesehen. Bei dieser Ausführungsform bildet das Werkzeugeingriffsmittel 119 folglich keine Applikationsöffnung 114 aus.

Bei der in den Figuren 1 und 2 gezeigten beispielhaften Ausführungsform eines Wirkstoffapplikationseinsatzes 10 wird die Wirkstoffkammer 12 auf der Vorderseite durch die Applikationsseite oder Applikationsstirnwand 13 und auf der Rückseite 16 durch den Deckel 20 ausgebildet. Zwischen der Applikationsseite 13 und dem Deckel 20 wird die Wirkstoffkammer 12 zudem durch eine umlaufende Wirkstoffkammerwand 18 begrenzt. Folglich wird die Wirkstoffkammer durch die als Stirnwand ausgebildete Applikationsseite 13, die als Wirkstoffkammerdeckel 20 ausgeführte Rückseite 16 und die umlaufende Wirkstoffkammerwand 18 definiert.

Die Wirkstoffkammerwand 18 wird in einem Abschnitt 17a/18a von der umlaufenden Außenwand 17 des Grundkörpers 11 ausgebildet. In einem sich daran anschließenden Abschnitt 18b zur Rückseite hin, ist die Wirkstoffkammerwand 18 hingegen getrennt von der umlaufenden Außenwand 17 ausgebildet. Aufgrund dieser getrennten Ausbildung ist die Wirkstoffkammerwand 18 in Bezug auf die umlaufende Außenwand 17 des Grundkörpers 11 innenliegend angeordnet. Dadurch befindet sich zwischen dem entsprechenden Abschnitt 17b der umlaufenden Außenwand 17 und dem Abschnitt 18b der Wirkstoffkammerwand 18 ein Spalt 17c.

Wie zum Beispiel der Figur 1 zu entnehmen ist, ist die umlaufende Außenwand 17 im Abschnitt 17b durch die Trennung von der Wirkstoffkammerwand 18 durchgehend ringförmig ausgebildet. Allerdings ist es auch möglich die umlaufende Außenwand 17 in diesem Bereich in Umfangsrichtung in Außenwandsektionen zu unterteilen, die durch Einschnitte in die umlaufende Außenwand 17 voneinander getrennt sind. Durch diese Außenwandsektionen lässt sich die Elastizität der Außenwand 17 noch weiter verringern. Dies ist insbesondere von Vorteil, wenn das Befestigungsmittel 15 als Snap-Fit ausgeführt ist. Bei einer solchen Ausführungsform kann zumindest ein Teil der Außenwandsektionen als Snap-Fit-Laschen mit einer Rastfunktion ausgeführt werden.

Durch die Trennung der umlaufenden Außenwand 17 des Wirkstoffapplikationseinsatzes 10 und der umlaufenden Wirkstoffkammerwand 18 in den Abschnitten 17b bzw. 18b und den dadurch hervorgerufenen ringförmigen Spalt 17c kann die Elastizität des Abschnitts 17b der umlaufenden Außenwand 17 eingestellt werden. Damit ist es möglich, auf die Stärke der Befestigung des Wirkstoffapplikationseinsatzes 10 in einem Implantat 30 Einfluss zu nehmen.

Wie in Figur 1 veranschaulicht, öffnet sich dieser Spalt 17c des Wirkstoffapplikationseinsatzes 10 auf der Seite der Applikationsseite 13 nach vorne. Es ist möglich, diesen Spalt 17c zusätzlich oder alternativ zu dem als Aussparung ausgeführten Werkzeugeingriffsmittel 19 zu verwenden. Im letzteren Fall kann auf die Aussparung bzw. das Durchgangsloch, das in Figur 1 in der Applikationsseite 13 dargestellt ist, verzichtet werden. Mit anderen Worten wird bei einer solchen Ausführungsform das Werkzeugeingriffsmittel 19 durch den Spalt 17c ausgebildet.

Bei den in den Figuren dargestellten Ausführungsformen eines Wirkstoffapplikationseinsatzes 10 ist die umlaufende Außenwand 17 mit einer Reibfläche 15a als Befestigungsmittel 15 versehen. Die Reibfläche 15a kann sich über die gesamte umlaufende Außenwand 17 erstrecken. Genauso ist es möglich, dass sich die Reibfläche 15a über einen Abschnitt der Außenwand 17 erstreckt, der sich in den Abschnitten 17a und/oder 17b befindet.

Die Reibfläche 15a kann als Querschnitterweiterung auf der umlaufenden Außenwand 17 des Grundkörpers 11 und/oder mit einer höheren Oberflächenrauigkeit versehen sein als die Oberfläche der umlaufenden Außenwand ohne Reibfläche 15a. Die Reibfläche kann zumindest oder nur auf dem weniger elastischen Abschnitt 17a vorgesehen sein. In diesem Abschnitt 17a ist es möglich, aufgrund der geringeren Elastizität bei der Reibfläche 15a eine höhere Reibkraft zu erzeugen, um den Wirkstoffapplikationseinsatz 10 in einem Implantat 30 zu befestigen

Durch Ausbilden der Reibfläche 15a im Abschnitt 17b der umlaufenden Außenwand 17 des Wirkstoffapplikationseinsatzes 10 kann die Elastizität 17b hingegen in einem breiteren Bereich gewählt werden, sodass der Wirkstoffapplikationseinsatz 10 beim Einsetzen in eine entsprechende Aussparung 31 eines Implantats 30 einfacher eingesetzt werden kann und danach in dieser Aussparung 31 fixiert ist. Zudem kann die Elastizität des Abschnitts 17a und/oder 17b so gewählt werden, dass auch eine Herausnahme des Wirkstoffapplikationseinsatzes 10 aus der Aussparung 31 eines Implantats 30 ohne übermäßigen Kraftaufwand möglich ist.

Die Elastizität wird vorzugsweise so gewählt, dass ein Einsetzen und/oder Herausnehmen per Hand möglich ist. Zumindest bei der Herausnahme wird dabei vorzugsweise eine Werkzeug verwendet, das in ein Werkzeugeingriffsmittel 19 des Wirkstoffapplikationseinsatzes eingreift.

Weiterhin wird durch die Trennung von Wirkstoffkammerwand 18 und Außenwand 17 des Grundkörpers 11 der Einfluss von Druckkräften, die durch das Einsetzen des Wirkstoffapplikationseinsatzes 10 in ein Implantat 30 entstehen, auf der Seite der Wirkstoffkammer 12 minimiert. Zum einen kann so einer möglichen Beschädigung der Wirkstoffkammer 12 vorgebeugt werden. Zudem kann auf der Applikationsseite 13 keine Verformung der Applikationsöffnungen 14 stattfinden und somit eine vorgesehene Dosierung nicht auf ungewünschte Weise beeinflusst werden. Dies ist vor allem bei Applikationsöffnungen 14 mit einer Größe in dem unteren Teil des oben angegebenen Bereichs von Vorteil.

Auch wenn der elastische Abschnitt 17b der umlaufenden Außenwand 17 bei der in den Figuren 1 bis 3 dargestellten beispielhaften Ausführungsform eines Wirkstoffapplikationseinsatzes 10 auf der Seite der Applikationsseite 13 angeordnet ist, ist es genauso möglich, diesen elastischeren Abschnitt 17b auf der Seite der Rückseite vorzusehen. Bei einer solchen Ausführungsform ist der ringförmige Spalt 17c im eingesetzten Zustand des Wirkstoffapplikationseinsatzes 10 dementsprechend dem Boden einer Aussparung 31 in einem Implantat 30 zugewandt.

Wie in den Figuren 1 bis 4 veranschaulicht, ist die umlaufende Außenfläche 17 des Wirkstoffapplikationseinsatzes 10, 110 zylindrisch. Neben der dargestellten kreiszylindrischen Form sind, wie oben bereits ausgeführt, auch andere zylindrische Formen vorstellbar. Allerdings vereinfacht die dargestellte zylindrische Form das Einsetzen des Wirkstoffapplikationseinsatzes 10, da dessen Orientierung um seine Längsachse, die sich von der Vorderseite zu der Rückseite des Wirkstoffapplikationseinsatzes 10 erstreckt, hierbei nicht berücksichtigt werden muss.

Wie ferner bei der Seitenansicht des Wirkstoffapplikationseinsatzes 10 in der Figur 2 erkennbar ist, ist die Rückseite 16 bzw. der Wirkstoffkammerdeckel 20 senkrecht zu der Längsachse des Wirkstoffapplikationseinsatzes 10 angeordnet. Dagegen weist die Applikationsseite 13 einen Winkel zur Längsachse auf. Durch diese zur Längsachse schräge Anordnung wird die Applikationsseite 13 der äußeren Kontur des Implantats 30 angepasst, in das der Wirkstoffapplikationseinsatz 10 einzusetzen ist. Je nach äußerer Kontur des Implantats 30 kann die Applikationsseite 13 auch eine komplexere Form, wie zum Beispiel eine gekrümmte Form, annehmen. Durch eine derartige Anpassung der Form und/oder Ausrichtung der Applikationsseite 13 an die äußere Kontur eines Implantats 30 kann insbesondere einer Verletzung umliegenden Gewebes vorgebeugt werden.

In Figur 4 ist lediglich eine sehr eingeschränkte Anpassung der Applikationsseite 13 einer zweiten Ausführungsform des Wirkstoffapplikationseinsatzes 110 an die Außenkontur des veranschaulichen Kniegelenkimplantats 30 ausgeführt worden. Weiterhin ist im Gegensatz zu der in den Figuren 1 bis 3 gezeigten Ausführungsform des Wirkstoffapplikationseinsatzes 10 die Applikationsseite 113 des Wirkstoffapplikationseinsatzes 110 mit ihren Applikationsöffnungen 114 als abnehmbarer Deckel 120 ausgeführt. Dadurch kann ein Ersetzen oder Auffüllen der Wirkstoffkammer erfolgen, ohne dass der Wirkstoffapplikationseinsatz 110 hierfür entnommen werden muss.

Die Wirkstoffapplikationseinsätze 10, 110 werden bevorzugt in dauerhafte Implantate 30 eingesetzt. Allerdings ist es genauso möglich, einen erfindungsgemäßen Wirkstoffapplikationseinsatz 10, 110 bei einer Interimsprothese zu verwenden. Allerdings besteht gerade bei dauerhaft bzw. endgültig eingesetzten Implantaten der Vorteil, das selbst im Falle einer Infektion, eine Explantation des Implantats im Allgemeinen vermieden wird.

Wird die vorliegende Erfindung bei einem Kniegelenkimplantat eingesetzt, so wird bevorzugt, einen erfindungsgemäßen Wirkstoffapplikationseinsatz auf der medialen und/oder lateralen Seite der Femurkomponente eines solchen Implantats vorzusehen. Bei solch einer Platzierung ist die Applikationsseite Weichteilgewebe zugewandt, wodurch sich der Wirkstoff schnell und zuverlässig in der Umgebung des Implantats ausbreitet.

### BEZUGSZEICHENLISTE

- 10, 110: Wirkstoffapplikationseinsatz
- 11: Grundkörper
- 12: Wirkstoffkammer
- 13, 113: Applikationsseite
- 14, 114: Applikationsöffnungen
- 15: Befestigungsmittel
- 15a: Reibfläche
- 16: Rückseite
- 16a: ringförmige Aussparung
- 17: umlaufende Außenwand
- 17a: Außenwandabschnitt als Kammerwand
- 17b: Außenwandabschnitt getrennt von der Wirkstoffkammerwand
- 17c: Spalt
- 18: umlaufende Wirkstoffkammerwand
- 18b: getrennter Abschnitt der Wirkstoffkammerwand
- 19, 119: Werkzeugeingriffsmittel
- 20, 120: Wirkstoffkammerdeckel
- 30: Implantat
- 31: Aussparung zur Aufnahme eines Wirkstoffapplikationseinsatzes

## Patentansprüche

1. Wirkstoffapplikationseinsatz (10, 110) für ein Implantat (30), insbesondere ein Gelenkimplantat, der aufweist:
- einen Grundkörper (11),
- eine in dem Grundkörper ausgebildete Wirkstoffkammer (12) ;
- eine Applikationsseite (13, 113), welche eine Vorderseite der Wirkstoffkammer ausbildet, wobei die Applikationsseite mehrere Applikationsöffnungen (14, 114) aufweist, und
- ein Befestigungsmittel (15), mit dem der Grundkörper abnehmbar in dem Implantat befestigbar ist,
**dadurch gekennzeichnet, dass** in einem Zustand, in dem der Grundkörper in das Implantat eingesetzt ist, die Applikationsseite relativ zu dem Implantat nach außen gewandt ist, sodass die Applikationsöffnungen die Wirkstoffkammer mit der Umgebung des Implantats verbinden.

2. Wirkstoffapplikationseinsatz (10, 110) nach Anspruch 1, bei dem
auf der zu der Applikationsseite (13, 113) gegenüberliegenden Seite eine Rückwand ausgebildet ist, welche eine Rückseite (16) der Wirkstoffkammer (12) ausbildet und in dem eingesetzten Zustand dem Implantat zugewandt ist.

3. Wirkstoffapplikationseinsatz (10) nach Anspruch 2, bei dem
zumindest ein Abschnitt der Rückwand auf der Rückseite (16) der Wirkstoffkammer (12) durch einen vorzugsweise wiederverschließbaren Deckel ausgebildet ist.

4. Wirkstoffapplikationseinsatz (10, 110) nach einem der vorstehenden Ansprüche, bei dem
der Grundkörper (11) zwischen der Vorderseite und der Rückseite (16) eine umlaufende Außenwand (17) aufweist, die zylindrisch, insbesondere mit einem kreisförmigen Querschnitt, ausgebildet ist und die Wirkstoffkammer (12) umgibt.

5. Wirkstoffapplikationseinsatz (10, 110) nach Anspruch 4, bei dem
zumindest ein Abschnitt der Außenseite der umlaufenden Außenwand (17) eine Reibfläche (15a) als Befestigungsmittel (15) ausbildet.

6. Wirkstoffapplikationseinsatz (10, 110) nach einem der Ansprüche 4 oder 5, bei dem
die umlaufende Außenwand (17) zumindest einen Abschnitt (17a) einer umlaufenden Wirkstoffkammerwand (18) zwischen der Applikationsseite (13, 113) und der Rückseite (16) der Wirkstoffkammer (12) ausbildet.

7. Wirkstoffapplikationseinsatz (10, 110) nach einem der Ansprüche 4 bis 6, bei dem
zumindest ein Abschnitt (17b) einer umlaufenden Wirkstoffkammerwand (18) zwischen der Applikationsseite (13, 113) und der Rückseite (16) der Wirkstoffkammer (12) getrennt von und innenliegend zu der umlaufenden Außenwand (17) des Grundkörpers (11) ausgebildet ist, sodass entlang dieses Abschnitts zwischen der Außenwand des Grundkörpers und der Wirkstoffkammerwand ein Spalt (17c) ausgebildet ist.

8. Wirkstoffapplikationseinsatz (10, 110) nach einem der vorstehenden Ansprüche, bei dem
die Applikationsseite (13, 113) mit einem Werkzeugeingriffsmittel (19, 119) zum Einsetzen und/oder Herausnehmen des Wirkstoffapplikationseinsatzes versehen ist.

9. Wirkstoffapplikationseinsatz (10, 110) nach Anspruch 8, bei dem
das Werkzeugeingriffsmittel (19, 119) als Aussparung ausgebildet ist, die vorzugsweise ein Innengewinde aufweist.

10. Wirkstoffapplikationseinsatz (10, 110) nach einem der vorstehenden Ansprüche, bei dem
die Applikationsseite (13, 113) mindestens 4, 6, 8 oder 10 und maximal 30 Applikationsöffnungen (14, 114) aufweist, die vorzugsweise gleichmäßig über die Applikationsseite verteilt sind.

11. Wirkstoffapplikationseinsatz (10, 110) nach einem der vorstehenden Ansprüche, bei dem
die Applikationsöffnungen (14, 114) eine Größe von mindestens 0,5 mm oder 1 mm und maximal 2 mm, 3 mm oder 4 mm aufweist.

12. Wirkstoffapplikationseinsatz (10, 110) nach einem der vorstehenden Ansprüche, bei dem
die Applikationsöffnungen (14, 114) kreisförmig, oval, rechtwinklig und/oder schlitzförmig vorgesehen sind.

13. Wirkstoffapplikationseinsatz (10, 110) nach einem der vorstehenden Ansprüche, mit mindestens einem Wirkstoff, insbesondere einem Antibiotikum, in Form eines die Wirkstoffkammer (12) zumindest teilweise ausfüllenden Feststoffs, Kugeln, Granulats und/oder Gels.

14. Implantat (30), insbesondere Gelenkimplantat, mit einem Wirkstoffapplikationseinsatz nach einem der vorstehenden Ansprüche und mindestens einer Aussparung, die für eine Aufnahme eines Wirkstoffapplikationseinsatzes (10, 110) nach einem der vorstehenden Ansprüche ausgeführt ist.

## Claims

1. Active substance application insert (10, 110) for an implant (30), in particular a joint implant, comprising:
- a main body (11),
- an active substance chamber (12) formed in the main body,
- an application side (13, 113), which forms a front face of the active substance chamber, wherein the application side has a plurality of application openings (14, 114), and
- a securing means (15) with which the main body can be detachably secured in the implant,
**characterized in that**, in a state in which the main body is inserted into the implant, the application side faces outwards relative to the implant, such that the application openings connect the active substance chamber to the surroundings of the implant.

2. Active substance application insert (10, 110) according to Claim 1, wherein a rear wall is formed on the side opposite the application side (13, 113), which rear wall forms a rear face (16) of the active substance chamber (12) and faces towards the implant in the inserted state.

3. Active substance application insert (10) according to Claim 2, wherein at least one section of the rear wall on the rear face (16) of the active substance chamber (12) is formed by a preferably re-closable cover.

4. Active substance application insert (10, 110) according to one of the preceding claims, wherein the main body (11) has a peripheral outer wall (17) between the front face and the rear face (16), which outer wall (17) is formed cylindrically, in particular with a circular cross section, and surrounds the active substance chamber (12).

5. Active substance application insert (10, 110) according to Claim 4, wherein at least one section of the outer side of the peripheral outer wall (17) forms a friction surface (15a) as securing means (15).

6. Active substance application insert (10, 110) according to either Claim 4 or 5, wherein the peripheral outer wall (17) forms at least one section (17a) of a peripheral active substance chamber wall (18) between the application side (13, 113) and the rear face (16) of the active substance chamber (12).

7. Active substance application insert (10, 110) according to one of Claims 4 to 6, wherein at least one section (17b) of a peripheral active substance chamber wall (18) between the application side (13, 113) and the rear face (16) of the active substance chamber (12) is formed separately from and to the inside of the peripheral outer wall (17) of the main body (11), such that a gap (17c) is formed along this section between the outer wall of the main body and the active substance chamber wall.

8. Active substance application insert (10, 110) according to one of the preceding claims, wherein the application side (13, 113) is provided with a tool-engaging means (19, 119) for inserting and/or removing the active substance application insert.

9. Active substance application insert (10, 110) according to Claim 8, wherein the tool-engaging means (19, 119) is formed as a recess, which preferably has an internal thread.

10. Active substance application insert (10, 110) according to one of the preceding claims, wherein the application side (13, 113) has at least 4, 6, 8 or 10 and a maximum of 30 application openings (14, 114), which are preferably distributed uniformly over the application side.

11. Active substance application insert (10, 110) according to one of the preceding claims, wherein the application openings (14, 114) have a minimum size of 0.5 mm or 1 mm and a maximum size of 2 mm, 3 mm or 4 mm.

12. Active substance application insert (10, 110) according to one of the preceding claims, wherein the application openings (14, 114) are circular, oval, rectangular and/or slit-shaped.

13. Active substance application insert (10, 110) according to one of the preceding claims, having at least one active substance, in particular an antibiotic, in the form of a solid, beads, a granulate and/or a gel, which fills the active substance chamber (12) at least in part.

14. Implant (30), in particular a joint implant, having an active substance application insert according to one of the preceding claims and at least one recess designed to receive an active substance application insert (10, 110) according to one of the preceding claims.

## Revendications

1. Insert d'application de substance active (10, 110) destiné à un implant (30), en particulier un implant articulaire, lequel insert comporte :
- un corps de base (11),
- une chambre de substance active (12) formée dans le corps de base,
- un côté d'application (13, 113) qui forme un côté avant de la chambre de substance active, le côté d'application comportant plusieurs ouvertures d'application (14, 114), et
- un moyen de fixation (15) permettant de fixer le corps de base de manière amovible dans l'implant,
**caractérisé en ce que**, dans un état dans lequel le corps de base est inséré dans l'implant, le côté d'application est dirigé vers l'extérieur par rapport à l'implant de sorte que les ouvertures d'application relient la chambre de substance active à l'environnement de l'implant.

2. Insert d'application de substance active (10, 110) selon la revendication 1, dans lequel
une paroi arrière, qui forme un côté arrière (16) de la chambre de substance active (12) et qui est dirigée vers l'implant à l'état inséré, est formée du côté opposé au côté d'application (13, 113).

3. Insert d'application de substance active (10) selon la revendication 2, dans lequel
au moins une portion de la paroi arrière du côté arrière (16) de la chambre de substance active (12) est formée par un couvercle de préférence refermable.

4. Insert d'application de substance active (10, 110) selon l'une des revendications précédentes, dans lequel
le corps de base (11) comporte entre le côté avant et le côté arrière (16) une paroi extérieure périphérique (17) qui est en forme de cylindre, en particulier de section transversale circulaire, et qui entoure la chambre de substance active (12).

5. Insert d'application de substance active (10, 110) selon la revendication 4, dans lequel
au moins une portion du côté extérieur de la paroi extérieure périphérique (17) forme une surface de frottement (15a) servant de moyen de fixation (15).

6. Insert d'application de substance active (10, 110) selon l'une des revendications 4 et 5, dans lequel
la paroi extérieure périphérique (17) forme au moins une portion (17a) d'une paroi de chambre de substance active périphérique (18) entre le côté d'application (13, 113) et le côté arrière (16) de la chambre de substance active (12).

7. Insert d'application de substance active (10, 110) selon l'une des revendications 4 à 6, dans lequel
au moins une portion (17b) d'une paroi de chambre de substance active périphérique (18) entre le côté d'application (13, 113) et le côté arrière (16) de la chambre de substance active (12) est formée séparément de la paroi extérieure périphérique (17) du corps de base (11) et du côté intérieur de celle-ci de sorte qu'un espace (17c) est ménagé le long de cette portion entre la paroi extérieure du corps de base et la paroi de chambre de substance active.

8. Insert d'application de substance active (10, 110) selon l'une des revendications précédentes, dans lequel
le côté d'application (13, 113) est pourvu d'un moyen d'engagement d'outil (19, 119) destiné à insérer et/ou retirer l'insert d'application de substance active.

9. Insert d'application de substance active (10, 110) selon la revendication 8, dans lequel
le moyen d'engagement d'outil (19, 119) est réalisé sous la forme d'un évidement qui comporte de préférence un filetage intérieur.

10. Insert d'application de substance active (10, 110) selon l'une des revendications précédentes, dans lequel
le côté d'application (13, 113) comporte au moins 4, 6, 8 ou 10 et au maximum 30 ouvertures d'application (14, 114) qui sont réparties de préférence régulièrement sur le côté d'application.

11. Insert d'application de substance active (10, 110) selon l'une des revendications précédentes, dans lequel
les ouvertures d'application (14, 114) ont une dimension d'au moins 0,5 mm ou 1 mm et au maximum de 2 mm, 3 mm ou 4 mm.

12. Insert d'application de substance active (10, 110) selon l'une des revendications précédentes, dans lequel
les ouvertures d'application (14, 114) sont circulaires, ovales, rectangulaires et/ou en forme de fente.

13. Insert d'application de substance active (10, 110) selon l'une des revendications précédentes, ledit insert comprenant au moins une substance active, notamment un antibiotique, sous forme d'une matière solide, de billes, de granulés et/ou de gel remplissant au moins partiellement la chambre de substance active (12).

14. Implant (30), notamment implant articulaire, comprenant un insert d'application de substance active selon l'une des revendications précédentes et au moins un évidement qui est destiné à recevoir un insert d'application de substance active (10, 110) selon l'une des revendications précédentes.
